# EUROPEAN PATENT APPLICATION

(11) **EP 4 393 486 A1**
(43) Date of publication of application: **03.07.2024**
(21) Application number: 22861775.9
(22) Date of filing: 24.08.2022
(51) Int. Cl.: A61K 31/05, A61P 3/00

(54) **COMPOSITIONS AND METHODS FOR PREVENTING OR REDUCING THE RISK OF METABOLIC SYNDROME**

(30) Priority: 25.08.2021 US 202163236875 P
(71) Applicant: García Sada, Fernando, San Pedro Garza García, Nuevo León 66220 (MX)
(72) Inventor: GOJÓN ZORRILLA, Gabriel, Nuevo León, 66220 (MX); GOJÓN ROMANILLOS, Gabriel, Nuevo León, 66220 (MX); GARCÍA ALANIS, Eduardo, Nuevo León, 66220 (MX)
(74) Representative: ABG Intellectual Property Law, S.L.
(86) International application number: PCT/MX2022/000001
(87) International publication number: WO 2023/027570

(57) **Abstract**

The invention provides a compositions and methods for use in the treatment and prevention onset or progression to metabolic syndrome in human or non-human mammal, the reduction or control of weight, levels of total cholesterol, triglycerides, glucose, and/or non-esterified fatty acids, comprising administering to a mammal a composition with an effective amount of a tyrphostin AG17 or any pharmaceutically acceptable salt thereof and pharmaceutically acceptable excipient including carrier, adjuvant, vehicle or mixtures thereof.

## Description

### Field of the Invention

The invention relates to compositions and methods of preventing or reducing the risk of metabolic syndrome and its associated complications.

### Background of the Invention

Metabolic syndrome is the name of a group of risk factors that occur together and that result in an increase in the risk of cardiovascular disorders, diabetes, blood clots, fatty liver disease and other health problems.

Metabolic disarrangement becomes a syndrome if the patient has any three of the following: (1) waist circumference more than 40 inches in men and 35 inches in women; (2) elevated triglycerides 150 milligrams per deciliter of blood (mg/dL) or greater; (3) reduced high-density lipoprotein cholesterol (HDL) less than 40 mg/dL in men or less than 50 mg/dL in women; (4) elevated fasting glucose of 100 mg/dL or greater; (5) blood pressure values of systolic 130 mmHg or higher and/or diastolic 85 mmHg or higher.

The underlying etiology of metabolic syndrome is extra weight, obesity, lack of physical activity, and genetic predisposition. The crux of the syndrome is a buildup of adipose tissue and tissue dysfunction that in turn leads to insulin resistance. Proinflammatory cytokines such as tumor necrosis factor, leptin, adiponectin, plasminogen activator inhibitor, and resistin, are released from the enlarged adipose tissue, which alters and impacts insulin handling adversely. Insulin resistance can be acquired or may be due to genetic disposition. Impairment of the signaling pathway, insulin receptor defects, and defective insulin secretion can all contribute towards insulin resistance. Over time, the culmination of this cause development of metabolic syndrome that presents as vascular and autonomic damage.

The distribution of body fat is also important, and it is known that upper body fat plays a strong role in developing insulin resistance. Fat accumulation can be intraperitoneal (visceral fat) or subcutaneous. Visceral fat may contribute to insulin resistance more strongly than subcutaneous fat. However, both are known to play a role in the development of the metabolic syndrome. In upper body obesity, high levels of nonesterified fatty acids are released from the adipose tissue causing lipid to accumulate in other parts of the body such as liver and muscle, further perpetuating insulin resistance.

The group risk factors include elevated blood pressure, elevated blood sugar, high triglyceride levels, low HDL cholesterol, and excess body fat around the waist or overweight.

Studies have shown that metabolic syndrome is heritable, indicating that genetic factors play a role in individual susceptibility to metabolic syndrome.

The cardiovascular disorders may be coronary artery disease, heart disease, heart attack and heart damage, as well as increased risk of stroke.

Diabetes is a chronic disease that occurs when the pancreas does not secrete enough insulin or when the body does not use the insulin it produces effectively. The effect of uncontrolled diabetes is hyperglycemia (high blood glucose), which, over time, severely damages many organs and systems, especially nerves and blood vessels. Can be presented in the form of: type 1 diabetes (insulin-dependent, juvenile o childhood-onset), type 2 diabetes (non-insulin-dependent), or gestational diabetes.

Tyrphostins are compounds to safely enhance mitochondrial respiration, through inhibition of the coupling between the electron transport and phosphorylation reactions and thus inhibit ATP synthesis, without affecting the respiratory chain and ATP synthase (H(+)-ATPase).

### Summary of the Invention

The invention provides compositions and methods for use preventing or reducing the risk of metabolic syndrome and its associated complications. More specifically, the invention includes administering a pharmaceutical composition to the subject comprising a tyrphostin AG17 or any pharmaceutically acceptable salt thereof and pharmaceutically acceptable excipient, including carrier, adjuvant, vehicle or mixtures thereof, without requiring a lifestyle modification or dietary intervention.

In an embodiment, the composition is adapted to provide to the subject tyrphostin AG17 or any pharmaceutically acceptable salt thereof, further required for a modified diet or exercise regime for the subject, or a combination thereof.

In an embodiment, the composition is adapted to provide to the mammal therapeutically effective amount of tyrphostin AG17 or any pharmaceutically acceptable salt thereof and pharmaceutically acceptable excipient.

### Detailed Description of the Invention

The invention provides compositions and methods of preventing or reducing the risk of metabolic syndrome and its associated complications. More specifically, the invention includes a pharmaceutical composition wherein the composition is adapted to provide to the subject tyrphostin AG17 or any pharmaceutically acceptable salt thereof.

### Definitions

As used herein, "a" or "an" means "at least one" or "one or more" unless otherwise indicated. In addition, the singular forms "a", "an", and "the" include plural referents unless the context clearly dictates otherwise.

As used herein, "about" refers to an amount .+-.25% of the recited value.

As used herein, "administration" or "administering" is meant a method of giving a dosage of a composition to a mammal so as to produce contact of the active ingredient(s) of said composition with its (their) site(s) of action.

As used herein, "composition" is meant a system comprising a substance or more than one substance described herein and pharmaceutically acceptable excipient including carrier, adjuvant, vehicle or mixtures thereof and manufactured or sold as part of a therapeutic or prophylactic regimen for the treatment of disease in a mammal or to promote and maintain general health.

As used herein, "effective amount" is meant an agent in an amount sufficient to effect beneficial or desired results, such as clinical results, and, as such, an "effective amount" depends upon the context in which it is being applied.

As used herein, "metabolic syndrome" is a group of risk factors that occur together and that result in an increase in the risk of cardiovascular disorders, diabetes, blood clots, fatty liver disease and other health problems.

As used herein, "overweight" is meant a subject whose body mass index (BMI) is between 25 kg/m² and 30 kg/m².

As used herein, "obese" is meant a subject whose body mass index (BMI) exceeds 30 kg/m².

As used herein, "preventing," as used herein, refers to prophylactic treatment or treatment that prevents one or more symptoms or development of a disease, disorder, or condition described herein.

As used herein, "subject" means a mammal, human or non-human animal (e.g., a dog, a cat, a monkey, a donkey, a horse, a cow, a pig, a mouse, a rat, etc.).

As used herein, "synergistic effect" is meant when two or more active agents (e.g., any of the compounds described herein) are administered together and the resulting effect is greater than the additive effect of each agent when administered singularly to a subject treated using the compositions of this invention.

As used herein, "therapeutic agent" or "prophylactic agent" is meant one or more active agents (e.g., any of the compounds described herein) formulated together in a single composition or one or more active agents (e.g., any of the compounds described herein) administered in combination to the subject.

As used herein, "treating" is meant subjecting a patient to a management regimen for the purpose of combating a disease or disorder and obtaining beneficial or desired results, such as clinical results.

Tyrphostin AG17 is a uncoupler of oxidative phosphorylation (Compound I):

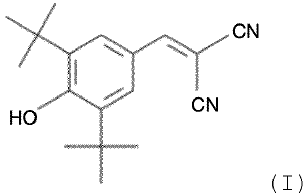

Compound I it's a yellow crystalline solid has a molecular weight of 282.4 g/mol, solubility of ~0.15 mg/ml in a 1:6 solution of DMSO:PBS (pH 7.2); ~12 mg/ml in EtOH; ~30 mg/ml in DMSO & DMF and oral LD50 of 87 mg/kg (rat).

In an embodiment, the present invention comprising the administration a pharmaceutical composition oral, wherein the composition is adapted to provide to the mammal a therapeutically effective amount of tyrphostin AG17 or any pharmaceutically acceptable salt thereof.

According to one aspect of the invention there is provided compositions and methods of preventing or reducing the risk of developing metabolic syndrome, such method comprising administering a pharmaceutical composition to human or non-human mammal an effective amount of a tyrphostin AG17 or any pharmaceutically acceptable salt thereof and a pharmaceutically acceptable excipient including carrier, adjuvant, vehicle or mixtures thereof.

According to a second aspect of the invention there is provided a pharmaceutical composition, wherein the composition is adapted to provide to the subject tyrphostin AG17 or any pharmaceutically acceptable salt thereof for use in a method for preventing or reducing or control the elevated blood pressure or elevated blood sugar or high triglyceride levels or low HDL cholesterol, or excess body fat around the waist and overweight.

The composition is adapted to provide to the subject tyrphostin AG17 or any pharmaceutically acceptable salt thereof, for the prevention of cardiovascular disorders (coronary artery disease, heart disease, heart attack and heart damage, as well as increased risk of stroke), diabetes (type 1 diabetes, type 2 diabetes or gestational diabetes), blood clots and other health problems.

That is, the present invention provides a pharmaceutical unit dosage form suitable for oral administration comprising about 0.1 to about 175 mg of Compound (I) or a dose equivalent to about 0.001 mg/Kg to about 1.75 mg/Kg and, a pharmaceutically acceptable excipient including carrier, adjuvant, vehicle or mixtures thereof.

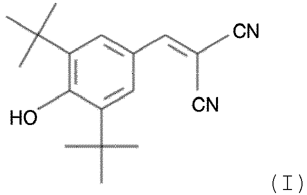

Compound (I), Tyrphostin AG17 is disclosed in European Patent Publication No. EP0322738, which describes the synthesis of Tyrphostin AG17 and related benzylidenemalonic acid derivatives.

In an embodiment, it is disclosed a method for preventing the onset or progression to metabolic syndrome in a human or non-human mammal, the reduction or control of weight, levels of total cholesterol, triglycerides, glucose, and/or non-esterified fatty acids, such method comprising administering to a mammal a composition, wherein the composition is adapted to provide to the mammal therapeutically effective amount of tyrphostin AG17 or any pharmaceutically acceptable salt thereof; more preferably of about 0.001 mg/Kg to about 1.75 mg/Kg of tyrphostin AG17 or any pharmaceutically acceptable salt thereof.

In an embodiment, it is disclosed a method for preventing the onset or progression to metabolic syndrome in a human or non-human mammal, lowering or control of weight, levels of total cholesterol, triglycerides, glucose, and/or non-esterified fatty acids, comprising administering to a mammal a composition, wherein the composition is adapted to provide to the subject with a dose of about 0.001 mg/Kg to about 0.1999 mg/Kg of tyrphostin AG17 or any pharmaceutically acceptable salt thereof. Alternatively, the composition is adapted to provide to the subject from about 0.1 mg to about 19.99 mg of tyrphostin AG17 or any pharmaceutically acceptable salt thereof.

In an embodiment, a composition is used for preventing the onset or progression to metabolic syndrome in a human or non-human mammal, the reduction or control of weight, levels of total cholesterol, triglycerides, glucose, and/or non-esterified fatty acids, wherein the composition is adapted to provide to the subject mammal a dose of about 0.20 mg/Kg to about 1.75 mg/Kg of tyrphostin AG17 or any pharmaceutically acceptable salt thereof; wherein the tyrphostin AG17 with a minimum purity of about 95% is used, more preferably with a minimum purity of about 98%. Alternatively, the composition is adapted to provide to the subject from about 20 mg to about 175 mg of tyrphostin AG17 or any pharmaceutically acceptable salt thereof.

The invention also features compositions including tyrphostin AG17 or pharmaceutically acceptable salt thereof which are is co-administered separately, sequentially, or simultaneously to a subject in need thereof with at least one L-carnitine or derivative or salt thereof, and pharmaceutically acceptable excipient including carrier, adjuvant, vehicle or mixtures thereof. Compounds of the invention include, but are not limited to L-carnitine, salts of L-carnitine, alkanoyl L-carnitines, and salts of alkanoyl L-carnitine.

In an embodiment, L-carnitine is present in an amount 2 mg to about 5000 mg. In certain embodiment L -carnitine is present in an amount of about 700 mg.

Suitable salts of L-carnitine and its derivatives include L-carnitine tartrate, L-carnitine chloride, L-carnitine bromide, L-carnitine acid aspartate, L-carnitine acid phosphate, L-carnitine fumarate, L-carnitine lactate, L-carnitine maleate, L-carnitine acid maleate, L-carnitine acid oxalate, L-carnitine acid sulfate, L-carnitine glucose phosphate, L-carnitine acid tartrate, L-carnitine iodate, L-carnitine aspartate, L-carnitine citrate, L-carnitine acid citrate, L-carnitine acid fumarate, L-carnitine glycerophosphate, L-carnitine mucate, L-carnitine orotate, L-carnitine oxalate, L-carnitine sulfate, L-carnitine trichloroacetate, L-carnitine trifluoroacetate, L-carnitine methanesulfonate, L-carnitine pamoate, L-carnitine acid pamoate, C₂₋₈ alkanoyl L-carnitines, C₂₋₈ alkanoyl L-carnitine chloride, C₂₋₈ alkanoyl L-carnitine bromide, C₂₋₈ alkanoyl L-carnitine orotate, C₂₋₈ alkanoyl L-carnitine acid aspartate, C₂₋₈ alkanoyl L-carnitine acid phosphate, C₂₋₈ alkanoyl L-carnitine fumarate, C₂₋₈ alkanoyl L-carnitine lactate, C₂₋₈ alkanoyl L-carnitine maleate, C₂₋₈ alkanoyl L-carnitine acid maleate, C₂₋₈ alkanoyl L-carnitine acid oxalate, C₂₋₈ alkanoyl L-carnitine acid sulfate, C₂₋₈ alkanoyl L-carnitine glucose phosphate, C₂₋₈ alkanoyl L-carnitine tartrate, C₂₋₈ alkanoyl L-carnitine acid tartrate, C₂₋₈ alkanoyl L-carnitine iodate, C₂₋₈ alkanoyl L-carnitine aspartate, C₂₋₈ alkanoyl L-carnitine citrate, C₂₋₈ alkanoyl L-carnitine acid citrate, C₂₋₈ alkanoyl L-carnitine acid fumarate, C₂₋₈ alkanoyl L-carnitine glycerophosphate, C₂₋₈ alkanoyl L-carnitine mucate, C₂₋₈ alkanoyl L-carnitine orotate, C₂₋₈ alkanoyl L-carnitine oxalate, C₂₋₈ alkanoyl L-carnitine sulfate, C₂₋₈ alkanoyl L-carnitine trichloroacetate, C₂₋₈ alkanoyl L-carnitine trifluoroacetate, C₂₋₈ alkanoyl L-carnitine methanesulfonate, C₂₋₈ alkanoyl L-carnitine pamoate, and C₂₋₈ alkanoyl L-carnitine acid pamoate.

Formulations for oral use include tablets containing the active ingredient(s) in a mixture with pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and anti-adhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

The tablets may be uncoated or may be coated by known techniques, optionally to delay disintegration and absorption in the gastrointestinal tract and thereby providing a sustained action over a longer period. The coating may be adapted to release the compound in a predetermined pattern (e.g., in order to achieve a controlled release formulation) or it may be adapted not to release the agent(s) until after passage of the stomach (enteric coating). The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols, and/or polyvinylpyrrolidone), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). Furthermore, a time delay material such as, e.g., glyceryl monostearate or glyceryl distearate, may be employed. The solid tablet compositions may include a coating adapted to protect the composition from unwanted chemical changes (e.g., chemical degradation prior to the release of the active substances). The compositions of the invention may be mixed together in the tablet, or may be partitioned. In one example, a first agent is contained on the inside of the tablet, and a second agent is on the outside, such that a substantial portion of the second agent is released prior to the release of the first agent.

Formulations for oral use may also be presented as chewable tablets, or as hard gelatin capsules wherein the active ingredient is mixed with an inert solid diluent (e.g., potato starch, lactose, microcrystalline cellulose, calcium carbonate, calcium phosphate, kaolin, and/or any pharmaceutically acceptable excipient or additive), or as soft gelatin capsules, wherein the active ingredient is mixed with water or an oil medium, for example, peanut oil, liquid paraffin, or olive oil. Formulations for oral use may also be presented as sachets.

Formulations for oral use may additionally be presented as extended release or prolonged release formulations/unit dosage forms. Powders and granulates may be prepared using the ingredients mentioned above under tablets and capsules in a conventional manner using, e.g., a mixer, a fluid bed apparatus, or spray drying equipment.

In one embodiment, the composition object of the present invention corresponds to doses of 0.001 mg/kg to about 0.1999 mg/kg of tyrphostin AG17 or to a dose between 0.20 mg/kg to about 1.75 mg/kg of tyrphostin, which can be adjusted depending on the patient condition in each of the associated parameters with the risk factors that occur together and that result in an increase in the risk of cardiovascular disorders, diabetes, blood clots, fatty liver disease, i.e. weight, food intake, fat free mass, fat mass, body mass index, glucose, cholesterol, triglycerides, alanine transaminase (ALT), aspartate aminotransferase (AST), high-density lipoprotein (HDL) or low-density lipoprotein (LDL).

### Examples

The following are examples of the methods and compositions provided herein. It is understood that various other embodiments may be practiced, given the general description provided above.

### Example 1. Evaluation toxicity.

To investigate the toxicity of tyrphostin AG17 the following doses were tested in mice, the animals were randomly divided into experimental groups corresponding to different doses of tyrphostin. Tyrphostin was administered intragastric via daily for 90 days, after 90 days, the mice were weighed and sacrificed by cervical dislocation.

The study groups and results are shown in the following table:

| **Group** | **n (mice)** | **Treatment (mg/Kg)** | Percentage of dead mice (%) |
|---|---|---|---|
| Control | 10 | 0 | 0 |
| Vehicle | 10 | 0 | 0 |
| T1 | 10 | 0.0001 | 0 |
| T2 | 10 | 0.000175 | 0 |
| T3 | 10 | 0.001 | 0 |
| T4 | 10 | 0.0175 | 0 |
| T5 | 10 | 0.175 | 0 |
| T6 | 10 | 0.18 | 0 |
| T7 | 10 | 0.76 | 0 |
| T8 | 10 | 1.70 | 0 |
| T9 | 10 | 1.75 | 0 |
| T10 | 10 | 5.50 | 10 |
| T11 | 10 | 17.50 | 10 |
| T12 | 10 | 25.00 | 0 |
| T13 | 10 | 28.00 | 10 |
| T14 | 10 | 40.00 | 10 |
| T15 | 10 | 50.00 | 20 |
| T16 | 10 | 55.50 | 20 |
| T17 | 10 | 100.00 | 40 |

In general, doses below 1.75 mg/kg showed no apparent signs of toxicity during the 90-day tyrphostin treatment; on the other hand, at doses of 5.50 mg/kg tyrphostin, mouse mortality was 10%, except at the dose of 25.00 mg/kg which did not show any dead mice; at doses of 50.00 and 55.50 mg/kg, mortality was 20% and at doses of 100.00 mg/kg mortality was 40%. For this first evaluation lot tyrphostin AG17 with a minimum purity of 95% is used.

In a second evaluation for the treatments with 5.50 mg/kg to 55.50 mg/kg of tyrphostin, using a lot tyrphostin AG17 with a minimum purity of 98% is used; the percentage of dead mice is reduced to 0% and in the case of the treatment with 100.00 mg/kg, the deaths are reduced to 10%.

### Example 2. Subacute toxicity.

Animal studies in male and female mice of the CD1 strain, and male rats of the Wistar strain were used.

During the quarantine period (15 days) they were kept in a chamber with conditions of (22 + 2°C, 55 + 5% humidity and a light/dark cycle of 12:12 h), with food and water *ad libitum.* Prior to the start of the experiment, the animals (mice and rats) were deprived of food for 4 h, and recovered 2 h after the administration of the drug.

Tyrphostin G17 + soybean oil + 0.9% saline solution + Tween 80 (50:50 + 1%), was administered intragastrically daily for 14 days through a stainless steel cannula.

The test animals were randomly divided into experimental groups corresponding to different doses of the drug, as shown in Table 2.

**Table 2. Assignment of experimental groups to determine subacute toxicity.**

| **Group** | **n (mice)** | **Treatment (mg/Kg)** | **Signs of subacute toxicity presented** |
|---|---|---|---|
| Control | 10 | 0 | No apparent signs |
| Vehicle | 10 | 0 | No apparent signs |
| AT1 | 10 | 0.175 | No apparent signs |
| AT2 | 10 | 0.55 | No apparent signs |
| AT3 | 10 | 1.75 | No apparent signs |
| AT4 | 10 | 5.50 | Lethargy (10%) |
| AT5 | 10 | 55.50 | Lethargy (20%) |
| AT6 | 10 | 100.00 | Death (40%) |

The doses of 5.50 to 55.50 mg/kg of tyrphostin showed a lethargic effect in the mice, however, such lethargy decreased in severity and frequency over the hours, until after 12 hours, the animal recovered. In addition, it only occurred in 10% and 20% of the populations of the AT4 and AT5 groups, respectively. In the case of the 100.00 mg/kg dose, it resulted in the death of 40% of the population.

### Example 3. Effectiveness of tyrphostin AG17 on metabolic syndrome risk factors.

Animal studies in high-fat diet-induced obesity model in a total 75 male C57BL/6/CIEABR mice 6 to 7 weeks of age.

The test system is based on the High-Fat Diet (HFD) induced obesity model. Among the various strains of mice, C57BL/6 are the most used because they exhibit abnormalities similar to human metabolic syndrome when fed this type of diet.

Tyrphostin G17 + injectable water + Tween 80 (1%), was administered intragastrically daily for 14 days through a stainless steel gastric tube.

During the quarantine period (10 days), all animals were given rodent food "PicoLab Mouse Diet 5058" food, later, they were given high fat food "Tso's High Fat Diet With Butter Fat" or "PicoLab Mouse Diet 5058" food, according to the assignment of the type of diet and treatment that each animal should consume (See table 3 and table 4).

**Table 3. Assignment of normal or hypercaloric diet consumption for 0.001 mg/Kg to 0.1999 mg/Kg.**

| **Group** | **n (mouse)** | **Diet** | **Treatment** |
|---|---|---|---|
| ED1 | 10 | Normal | N/A |
| ED2 | 10 | HFD | N/A |
| ED3 | 10 | HFD | Vehicle |
| ED4 | 10 | HFD | 0.001 |
| ED5 | 10 | Normal | 0.001 |
| ED6 | 10 | HFD | 0.0175 |
| ED7 | 10 | Normal | 0.0175 |
| ED8 | 10 | HFD | 0.175 |
| ED9 | 10 | Normal | 0.175 |
| ED10 | 10 | HFD | 0.18 |
| ED11 | 10 | Normal | 0.18 |
| ED12 | 10 | HFD | 0.1999 |
| ED13 | 10 | Normal | 0.1999 |

At the end of the induction period, the administration of the different treatments began. The test product, vehicle, and controls were administered daily for two weeks.

Administration was orally using curved stainless steel gastric tubes (18G x 1.5) previously sanitized; administration was performed at 11:00 ± 1 h.

For the dose 0.20 mg/Kg to about 100 mg/Kg, lot tyrphostin AG17 with a minimum purity of 95% is used.

For each treatment, different parameters were quantified to determine their decrease regarding the treatment applied to each group: weight loss (%), feed consumption (%), fat free mass (g), fat mass (g), body mass index, glucose (%), cholesterol (%) and triglycerides (%).

**Table 4. Assignment of normal or hypercaloric diet consumption for 0.20 mg/Kg to about 100 mg/Kg.**

| **Group** | **n (mouse)** | **Diet** | **Treatment** |
|---|---|---|---|
| C1 | 10 | Normal | N/A |
| C2 | 10 | HFD | N/A |
| C3 | 10 | HFD | Vehicle |
| EH4 | 10 | HFD | 0.20 |
| EH5 | 10 | Normal | 0.20 |
| EH6 | 10 | HFD | 0.76 |
| EH7 | 10 | Normal | 0.76 |
| EH8 | 10 | HFD | 1.75 |
| EH9 | 10 | Normal | 1.75 |
| EH10 | 10 | HFD | 5.50 |
| EH11 | 10 | Normal | 5.50 |
| EH12 | 10 | HFD | 25.00 |
| EH13 | 10 | Normal | 25.00 |
| EH14 | 10 | HFD | 55.50 |
| EH15 | 10 | Normal | 55.50 |

### Results

Results show that management oral gavage acute tyrphostin AG17 caused an increase in mortality at the doses of 28, 40 and 55.5 mg/kg, with no lethal effect observed for the doses of 1.75, 5.5 and 17.5 mg/kg. Findings of behavioral alterations (inhibition in locomotion and piloerection) were reported in animals administered with these doses: 1.75, 5.5 and 17.5 mg/kg., Reporting an LD50 = 33.3 mg/kg. The doses reported for the evaluation of the antiobesity efficacy, oral gavage, of tyrphostin AG17, were 0.175, 0.0175 or 0.00175 mg/kg administered daily for 2 weeks, without alteration of plasma biochemical markers.

The results of the parameters related to metabolic syndrome for treatment with 0.001 mg/kg to 0.1999 mg/kg of tyrphostin are presented below, indicating the reduction of the parameter regarding each treatment:

**Table 5. Results of treatment evaluation with tyrphostin from 0.001 mg/kg to 0.1999 mg/kg.**

| **Group** | **Weight loss (%)** | **Food consumption (%)** | **Fat free mass (g)** | **Fat mass (g)** | **Body mass index** |
|---|---|---|---|---|---|
| C1 | 0.97 | 0.00 | 1.50 | 0.84 | -1.20 |
| C2 | -26.10 | -14.28 | -9.24 | -7.00 | -4.63 |
| C3 | -31.51 | 1.14 | 1.33 | -5.30 | -6.02 |
| ED4 | 3.17 | -4.21 | -2.69 | -0.54 | 1.04 |
| ED5 | 4.01 | 1.05 | -4.02 | 1.28 | 1.69 |
| ED6 | 6.19 | 13.04 | 1.22 | -0.31 | 2.03 |
| ED7 | 8.40 | 17.39 | 3.21 | 2.59 | 2.73 |
| ED8 | 17.16 | 25.00 | 2.34 | 1.28 | 4.41 |
| ED9 | 22.13 | 20.83 | 3.98 | 3.56 | 4.30 |
| ED10 | 16.56 | 21.74 | 3.32 | -0.10 | 3.30 |
| ED11 | 20.88 | 22.92 | 5.11 | 4.92 | 4.22 |
| ED12 | 16.24 | 22.27 | 3.41 | 0.36 | 3.35 |
| ED13 | 18.21 | 23.11 | 5.39 | 6.02 | 3.91 |

| **Group** | **Glucose (%)** | **Cholesterol (%)** | **Triglycerides (%)** |
|---|---|---|---|
| C1 | 0.21 | -0.57 | -0.87 |
| C2 | -1.91 | -2.06 | -18.26 |
| C3 | -1.42 | -2.33 | -12.17 |
| ED4 | 0.75 | -0.03 | 4.35 |
| ED5 | 1.33 | 0.10 | 7.83 |
| ED6 | 0.92 | 0.04 | 17.39 |
| ED7 | 1.24 | 0.44 | 20.00 |
| ED8 | 1.36 | 0.65 | 24.35 |
| ED9 | 1.93 | 1.30 | 25.22 |
| ED10 | 1.49 | 1.10 | 32.17 |
| ED11 | 2.31 | 1.27 | 29.57 |
| ED12 | 1.40 | 0.94 | 30.54 |
| ED13 | 2.11 | 2.44 | 30.43 |

Negative values indicate that the parameter evaluated increased rather than decreased after treatment.

The results of the parameters related to metabolic syndrome for the treatment with 0.20 mg/kg to about 55.50 mg/kg of tyrphostin are presented below, indicating the reduction of the parameter regarding each treatment:

**Table 6. Results of treatment evaluation with tyrphostin from 0.20 mg/Kg to about 100 mg/Kg.**

| **Group** | **Weight loss (%)** | **Food consumption (%)** | **Fat free mass (g)** | **Fat mass (g)** | **Body mass index** |
|---|---|---|---|---|---|
| C1 | 0.97 | 0.00 | 1.50 | 0.84 | -1.20 |
| C2 | -26.10 | -14.28 | -9.24 | -7.00 | -4.63 |
| C3 | -31.51 | 1.14 | 1.33 | -5.30 | -6.02 |
| EH4 | 20.88 | 18.18 | 3.44 | 0.39 | 3.34 |
| EH5 | 20.97 | 22.07 | 4.92 | 5.99 | 4.05 |
| EH6 | 14.92 | 23.81 | 2.39 | 1.21 | 2.23 |
| EH7 | 17.21 | 30.10 | 3.94 | 7.52 | 3.43 |
| EH8 | 18.14 | 33.11 | -0.12 | 1.05 | 2.50 |
| EH9 | 13.88 | 35.18 | 1.04 | 6.98 | 3.40 |
| EH10 | 7.83 | 38.09 | -0.04 | 1.28 | 3.30 |
| EH11 | 8.87 | 47.82 | 0.42 | 5.41 | 4.22 |
| EH12 | 9.27 | 37.27 | -0.13 | 1.20 | 3.35 |
| EH13 | 7.30 | 40.91 | -0.10 | 5.58 | 3.91 |
| EH14 | 3.49 | 33.12 | -0.12 | 1.06 | 2.54 |
| EH15 | 4.44 | 41.37 | -0.09 | 5.01 | 2.99 |

| **Group** | **Glucose (%)** | **Cholesterol (%)** | **Triglycerides (%)** |
|---|---|---|---|
| C1 | 0.21 | -0.57 | -0.87 |
| C2 | -1.91 | -2.06 | -18.26 |
| C3 | -1.42 | -2.33 | -12.17 |
| EH4 | 1.43 | 1.13 | 31.02 |
| EH5 | 2.15 | 2.48 | 30.75 |
| EH6 | 2.05 | 1.49 | 33.12 |
| EH7 | 2.04 | 3.38 | 31.74 |
| EH8 | 2.19 | 2.02 | 33.45 |
| EH9 | 2.43 | 3.43 | 32.42 |
| EH10 | 3.11 | 2.36 | 34.17 |
| EH11 | 2.03 | 3.44 | 32.56 |
| EH12 | 4.20 | 2.49 | 37.21 |
| EH13 | 2.10 | 3.45 | 32.45 |
| EH14 | 4.38 | 2.33 | 37.84 |
| EH15 | 1.91 | 3.58 | 33.32 |

For the doses 0.001 mg/Kg to about 0.1999 mg/Kg of tyrphostin AG17, the parameters evaluated showed a considerable decrease in weight and in the concentration of triglycerides, maintaining the levels of HDL and glucose; with low presence of liver damage or toxic effects.

For the doses 0.20 mg/Kg to about 1.75 mg/Kg of tyrphostin AG17, the parameters evaluated showed a decrease in weight and in the concentration of triglycerides, however, HDL levels were reduced and glucose levels were maintained; the use of high purity tyrphostin (<98%) reduced the effect on the hepatic liver as well as the toxic effects.

The pharmacokinetic parameters following administration of a 10 mg dose of tyrphostin are presented below.

**Table 7. Pharmacokinetic parameters following administration of a 10 mg dose of tyrphostin.**

| **Parameter** | **Mean** | **CV (%)** | **Minimum** | **Maximum** | **Median** | **Geometric Mean** |
|---|---|---|---|---|---|---|
| **T_{máx} (h)** | 2.250 | 31.4 27 | 1.000 | 3.000 | 2.500 | 2.129 |
| **C_{máx} (ng/mL)** | 625.110 | 18.2 75 | 429.888 | 791.808 | 607.361 | 615.683 |
| **kₑ (h⁻¹)** | 0.009 | 19.2 78 | 0.006 | 0.011 | 0.009 | 0.009 |
| **t_{1/2} (h)** | 82.203 | 22.5 39 | 63.328 | 119.566 | 75.151 | 80.573 |
| **ABC₀₋ₜ (h*ng/m L)** | 54054.9 85 | 25.4 81 | 35144.7 01 | 74122.0 98 | 55047.4 35 | 52426.1 91 |
| **ABC_{0-inf} (h*ng/m L)** | 54408.8 88 | 25.6 42 | 35215.0 26 | 74268.7 36 | 55369.5 50 | 52745.3 60 |
| **TMR (h)** | 115.354 | 22.2 65 | 87.649 | 161.116 | 105.242 | 113.013 |
| **ABC_{Ext} (%)** | 0.604 | 97.2 93 | 0.190 | 1.908 | 0.420 | 0.434 |
| **Vdₐₚₐ (L)** | 22.537 | 22.1 08 | 14.702 | 30.535 | 22.494 | 22.038 |
| **Clₐₚₐ (L/ h)** | 0.196 | 28.4 18 | 0.135 | 0.284 | 0.181 | 0.190 |

## Claims

1. A composition for use in the treatment and prevention of the onset or progression of metabolic syndrome in a human or non-human mammal, the reduction or control of weight, levels of total cholesterol, triglycerides, glucose, and/or non-esterified fatty acids, wherein the composition comprises tyrphostin AG17 or any pharmaceutically acceptable salt thereof and a pharmaceutically acceptable excipient.

2. A composition for use according to claim 1, wherein the composition is adapted to provide to the mammal a therapeutically effective amount of tyrphostin AG17 or any pharmaceutically acceptable salt thereof.

3. A composition according to claims 1 and 2, wherein said composition is adapted to provide of about 0.001 mg/Kg to about 1.75 mg/Kg of tyrphostin AG17 or any pharmaceutically acceptable salt thereof.

4. A composition according to claims 1 and 2, wherein said composition is adapted to provide of about 0.1 mg to about 175 mg of tyrphostin AG17 or any pharmaceutically acceptable salt thereof.

5. A composition according to claims 1 and 2, wherein said composition is adapted to provide of about 0.001 mg/Kg to about 0.1999 mg/Kg of tyrphostin AG17 or any pharmaceutically acceptable salt thereof.

6. A composition according to claims 1 and 2, wherein said composition is adapted to provide of about 0.1 mg to about 19.99 mg of tyrphostin AG17 or any pharmaceutically acceptable salt thereof.

7. A composition according to claims 1 and 2, wherein said composition is adapted to provide of about 0.20 mg/Kg to about 1.75 mg/Kg of tyrphostin AG17 or any pharmaceutically acceptable salt thereof; and wherein the tyrphostin AG17 or the pharmaceutically acceptable salt is of at least about 95% purity.

8. A composition according to claims 1 and 2, wherein said composition is adapted to provide of about 20 mg to about 175 mg of tyrphostin AG17 or any pharmaceutically acceptable salt thereof; and wherein the tyrphostin AG17 or the pharmaceutically acceptable salt thereof is of at least about 95% purity.

9. A composition according to claims 1 and 2, wherein said composition further comprises L-carnitine or a derivative or a salt thereof and pharmaceutically acceptable excipient.

10. The method for treatment and prevention of the onset or progression of metabolic syndrome in a human or non-human mammal, the reduction or control of weight, levels of total cholesterol, triglycerides, glucose, and/or non-esterified fatty acids, wherein said method comprises administering to the mammal, a composition according to claim 1.

11. The method according to claim 10, wherein said method comprises administering to the mammal the composition of claim 3.

12. The method according to claim 10, wherein said method comprises administering to the mammal the composition of claim 4.

13. The method according to claim 10, wherein said method comprises administering to the mammal the composition of claim 5.

14. The method according to claim 10, wherein said method comprises administering to the mammal the composition of claim 6.

15. The method according to claim 10, wherein said method comprises administering to the mammal the composition of claim 7.

16. The method according to claim 10, wherein said method comprises administering to the mammal the composition of claim 8.

17. The method according to claim 10, wherein said method comprises administering to the mammal the composition of claims 1 and 2, and further is co-administered separately, sequentially, or simultaneously to a subject in need thereof with at least one of L-carnitine or derivative or salt thereof.

18. The method according to claim 10, wherein the method comprises a lifestyle modification and/or dietary intervention for the subject.
